# EUROPÄISCHE PATENTSCHRIFT

(11) **EP 2 977 373 B1**
(45) Veröffentlichungstag und Bekanntmachung des Hinweises auf die Patenterteilung: **25.10.2017**
(21) Anmeldenummer: 15178032.7
(22) Anmeldetag: 23.07.2015
(51) Int. Cl.: C07D 307/12, C07C 67/00, C07B 41/12, C07B 53/00, C07C 69/75, C07C 69/74, C07C 69/753, C07C 69/24, C07C 69/612, C07C 69/616, C07C 69/65, C07C 69/40

(54) **VERFAHREN ZUR KATALYTISCHEN HERSTELLUNG VON ESTERN**
METHOD FOR THE CATALYTIC PRODUCTION OF ESTERS
PROCEDE DE FABRICATION CATALYTIQUE D'ESTERS

(30) Priorität: 24.07.2014 DE 102014214606
(43) Veröffentlichungstag der Anmeldung: 27.01.2016
(73) Patentinhaber: Evonik Degussa GmbH, 45128 Essen (DE)
(72) Erfinder: Beller, Matthias, 18211 Nienhagen (DE); Jackstell, Ralf, 27478 Cuxhaven Altenwalde (DE); Liu, Qiang, 300161 Hedong District Tianjin (CN); Wu, Lipeng, 055150 City Xingtai (CN)

(56) Entgegenhaltungen:
- US-A- 3 646 116
- QIANG LIU ET AL: "Ruthenium-Catalyzed Alkoxycarbonylation of Alkenes with Paraformaldehyde as a Carbon Monoxide Substitute", CHEMCATCHEM, Bd. 6, Nr. 10, 2. September 2014 (2014-09-02), Seiten 2805-2809, XP055220035, DE ISSN: 1867-3880, DOI: 10.1002/cctc.201402304
- QIANG LIU ET AL: "Regioselective Pd-Catalyzed Methoxycarbonylation of Alkenes Using both Paraformaldehyde and Methanol as CO Surrogates", ANGEWANDTE CHEMIE INTERNATIONAL EDITION, Bd. 54, Nr. 15, 7. April 2015 (2015-04-07) , Seiten 4493-4497, XP055219963, DE ISSN: 1433-7851, DOI: 10.1002/anie.201410764
- LIPENG WU ET AL: "Carbonylations of Alkenes with CO Surrogates", ANGEWANDTE CHEMIE INTERNATIONAL EDITION, Bd. 53, Nr. 25, 27. Mai 2014 (2014-05-27), Seiten 6310-6320, XP055219994, DE ISSN: 1433-7851, DOI: 10.1002/anie.201400793
- LIPENG WU ET AL: "Ruthenium-catalysed alkoxycarbonylation of alkenes with carbon dioxide", NATURE COMMUNICATIONS, Bd. 5, 11. Februar 2014 (2014-02-11), Seiten 1-6, XP055152708, DOI: 10.1038/ncomms4091
- HIDEYUKI KONISHI ET AL: "Remarkable Improvement Achieved by Imidazole Derivatives in Ruthenium-Catalyzed Hydroesterification of Alkenes Using Formates", ORGANIC LETTERS, Bd. 14, Nr. 18, 21. September 2012 (2012-09-21), Seiten 4722-4725, XP055219873, US ISSN: 1523-7060, DOI: 10.1021/ol301850y
- Anonymous: "alkenes" In: "IUPAC Compendium of Chemical Terminology", 24. Februar 2014 (2014-02-24), IUPAC, Research Triagle Park, NC, XP055219897, ISBN: 978-0-9678550-9-7 DOI: 10.1351/goldbook.A00224, * das ganze Dokument *

## Beschreibung

Die Erfindung betrifft ein Verfahren zur katalytischen Herstellung von Estern durch Alkoxycabonylierung , wobei mindestens ein Alkens mit Formaldehyd und mindestens einem Alkohol in Gegenwart eines Rutheniumkatalysators umgesetzt wird. Die Umsetzung erfolgt in Abwesenheit von Säuren als Cokatalysatoren.

Die Estergruppierung ist eine weitverbreitete und wichtige funktionelle Gruppe in der Chemie. Ester finden in der chemischen Industrie verschiedene Verwendungsmöglichkeiten. Aliphatische Ester können als Detergenzien z.B. als Carboxylate oder nach Hydrierung als Alkohole Anwendung finden.

Heutzutage stellt die Alkoxycarbonylierung von leicht verfügbaren Olefinen und Alkoholen sowie Kohlenmonoxid einen einfachen Syntheseweg für industriell bedeutsame Carbonsäureester dar. Unter Verwendung von Palladiumkatalysatoren ist insbesondere die Methoxycabonylierung von Bedeutung (C. Jimenez Rodriguez, D. F. Foster, G. R. Eastham, D. J. Cole-Hamilton, Chemical Communications 2004, 0, 1720-1721). Ein großer Nachteil des Verfahrens ist die Verwendung des Gases Kohlenmonoxid, das toxisch ist sowie leicht entflammbar und deshalb auch schwierig in großen Mengen zu transportieren. Bemerkenswerter Weise hat Lucite International (US) ein neues Verfahren zur Herstellung von mehr als 300000 t Methylpropionsäuremethylester (eine Vorstufe für das wichtige Monomer Methylmethacrylat) pro Jahr auf der Grundlage von palladiumkatalysierten Alkoxycarbonylierungen entwickelt und in die Anwendung gebracht. In diesen Klassen von Reaktionen sind jedoch große Mengen an Brönstedtsäuren als Cokatalysator (z. B. Methansulfonsäure oder Schwefelsäure) zur Erzeugung der katalytisch aktiven Pd-Hydridspecies notwendig. Abgesehen von ökonomischen Faktoren kann unter diesen Umständen auch die Korrosion der chemischen Reaktoren zu einem Problem werden.

Weiterhin ist der Einsatz von Formiaten als CO Erzatzquellen in katalytischen Alkoxycarbonylierungen bekannt (H. Konishi, T. Ueda, T. Muto, K. Manabe, Org. Lett. 2012, 14, 4722-4725). Die verwendeten Alkylformiate (HCOOR) sind aber in ihrer Anwendung aufgrund ihrer vorgegebenen R Gruppe limitiert, was eine breite Anwendbarkeit zur Synthese diverser Ester einschränkt. Außerdem sind im Allgemeinen harsche Reaktionsbedingungen (z. B. hohe Temperaturen), hohe Katalysatorbeladung und zusätzlicher Druck für diese Reaktionen nachteilig. Durch die chelatisierende Hydroveresterung von Olefinen mit 2-Pyridylformiat konnte die Reaktionstemperatur auf 135 °C gesenkt werden, aber die allgemeine Anwendbarkeit ist durch die dirigierende Gruppe eingeschränkt. Darüber hinaus wird weiterhin Methylformiat verwendet, welches industriell aus Methanol, Kohlenmonoxid und einer starken Base hergestellt wird. Dementsprechend ist die Carbonylierung mit Methylformiat nicht wirklich eine kohlenmonoxidfreie Reaktion.

Bekannt ist auch die Verwendung von Kohlendioxid als C1-Baustein bei der Alkoxycarbonylierung von Alkenen unter Verwendung von Rutheniumkatalysatoren. Jedoch werden spezielle Hochdruckreaktoren für die Umsetzung benötigt Wu, L.; Liu, Q; Fleischer, I.; Jackstell, R.; Beller, M. Nat. Commun. 4:3091 doi: 10.1038/ncomms4091 (2013).

Die Aufgabe der Erfindung besteht daher darin, ein einfaches Verfahren zur katalytischen Herstellung von diversen Estern bereitzustellen, das es ermöglicht, das Reaktionsprodukt in guten Ausbeuten kostengünstig herzustellen, wobei die Entstehung von Nebenprodukten möglichst gering gehalten werden soll sowie ein Einsatz von säurehaltigen Cokatalysatoren und von Gasen wie CO oder CO₂ vermieden werden soll.

Die Aufgabe wird gelöst durch ein Verfahren zur katalytischen Herstellung von Estern durch Alkoxycarbonylierung,
dadurch gekennzeichnet, dass mindestens ein Alken mit Formaldehyd und mindestens einem Alkohol in Gegenwart eines Rutheniumkatalysators umgesetzt wird,
wobei Alkene, die im erfindungsgemäßen Verfahren als Ausgangsmaterialien fungieren, können einfach oder mehrfach ungesättigte lineare n-Alkene oder verzweigte Isoalkene, Cycloalkene und aromatische Alkene mit einer Kohlenstoffzahl bevorzugt von 2 bis 40 sein, die gegebenenfalls mit Ester-, Aryl-, Nitro-, Hydroxyl-, Nitril-, Ethergruppen oder Halogenatomen substituiert sind;
wobei Formaldehyde Paraformaldehyd der allgemeinen Formel [CH₂O]ₙ bedeutet, wobei n = 1 bis 200 sein kann, und wobei das Paraformaldehyd festes Formaldehyd-Polymer bezeichnet, das eine Mischung von kurzkettigen, linearen Poly(oxymethylen)en enthält,
und der Katalysator ein Komplex aus Ru₃(CO)₁₂ oder Ru(Me-allyl)₂(COD) ist, gegebenenfalls in Kombination mit einem Halogenid und/oder einer trivalenten organischen Phosphorverbindung.

Formaldehyd im Sinne der Erfindung bedeutet Paraformaldehyd der allgemeinen Formel [CH₂O]ₙ, wobei n = 1 bis 200 sein kann. Als Paraformaldehyd wird das feste Formaldehyd-Polymer bezeichnet, das eine Mischung von kurzkettigen, linearen Poly(oxymethylen)en enthält und das z.B. beim Eindampfen von Formaldehyd-Lösung im Vakuum erhältlich ist. Beim Erhitzen auf ca. 100 °C zerfällt Paraformaldehyd reversibel oder durch Säure-Einwirkung in monomeres Formaldehyd.

Alkene, die im erfindungsgemäßen Verfahren als Ausgangsmaterialien fungieren, können einfach oder mehrfach ungesättigte lineare n-Alkene oder verzweigte Isoalkene, Cycloalkene und aromatische Alkene mit einer Kohlenstoffzahl bevorzugt von 2 bis 40 sein, die gegebenenfalls substituiert sind. Vorzugsweise werden Alkene mit 2 bis zu 20 C-Atomen eingesetzt. Als Substituenten kommen Ester-, Aryl-, Nitro-, Hydroxyl-, Nitril-, Ethergruppen oder Halogenatome in Frage.
Beispielsweise seien Ethen, Propylen, Buten, Isobuten, Penten, Hexen, Hepten, 1- und 2-Octen, Octa-1,7-dien, Cyclopenten, Cyclohexen, Bicyclo[2.2.1]hept-2-en, Styren, Allylbenzol, Isopropenylbenzol, 1-Isopropenyl-4-methylbenzol, 1-Chlor-4-isopropenylbenzol, 1-Fluor-4-isopropenylbenzol, 2-Isopropenylnaphthalin, Methylacrylat, Methylmethacrylat genannt. Weiterhin sind auch Gemische verschiedener Alkene einsetzbar.

Die Umsetzung erfolgt vorzugsweise mit linearen oder verzweigten aliphatischen Alkoholen, die ebenfalls mit den o.g. Substituenten subsituiert sein können. Vorzugsweise kommen Alkohole mit 1 bis 12 Kohlenstoffatomen zum Einsatz. Dazu gehören z.B. Methanol, Ethanol, Propanole, Butanole, Pentanole, Hexanole, Heptanole, Oktanole, Phenylmethanol, Phenylethanol, Anisylalkohol, 4-Fluorbenzylalkohol und Furfurylalkohol.

Das erfindungsgemäße Verfahren erfolgt in Lösung gegebenenfalls unter Zugabe von weiteren organischen Lösungsmitteln. Bevorzugt handelt es sich bei den Lösungsmitteln um protische und aprotische Lösungsmittel. Als protische Lösungsmittel fungieren z.B. die Alkohole. Als aprotische fungieren z.B. Amide, wie N-Methyl-pyrrolidon (NMP), Dimethylacetamid, Formamid, Methylformamid und Dimethylformamid.
Weitere aprotische unpolare Lösungsmittel können sein Alkane, z.B. Octan; Ether, z.B. Dioxan oder Tetrahydrofuran; als aprotische polare Lösungsmittel kommen z.B. Ketone, wie Aceton zum Einsatz. Des Weiteren können auch Propylencarbonat, Sulfolan, DMSO, Diglyme, Triglyme und Tetraglyme eingesetzt werden; ebenso aromatische Lösungsmittel wie Toluol, Benzol oder Xylol. Alle Lösungsmittel können einzeln oder im Gemisch untereinander mit anderen Lösungsmitteln eingesetzt werden.
Besonders bevorzugt wird NMP als Lösungsmittel im erfindungsgemäßen Verfahren verwendet.

Der beim erfindungsgemäßen Verfahren verwendete Rutheniumkatalysator wird bevorzugt in Form einer Vorläuferverbindung als Rutheniumkomplexverbindung eingesetzt, die gegebenenfalls durch einen oder mehrere weitere Liganden koordiniert ist.
Die Liganden sind bevorzugt ausgewählt aus der Gruppe umfassend CO, Halogenide, Tetrafluoroborat, Amide, Oxide, Carbanionen wie Methallyl, Cyclopentadienyl-, Cyclooctadienylliganden (Cp*), Carboxylate, Hydride, Sulfate und/oder trivalente organische Phosphorverbindungen.

In einer bevorzugten Ausführungsvariante des Verfahrens weist der Katalysator mindestens Triruthenium-dodecacarbonyl [Ru₃(CO)₁₂] oder Bis(2-methylallyl)(1,5-cyclooctadien) ruthenium(II) [Ru(Me-allyl)₂(COD)] auf, bevorzugt in Kombination mit einem Halogenid und/oder einer trivalenten organischen Phosphorverbindung.

Phosphorverbindungen und/oder andere Liganden können gegebenenfalls in einer Vorreaktion mit dem Rutheniumkomplex kombiniert werden oder sie werden in situ zur Reaktion gegeben.

Die Phosphorverbindungen sind z.B. monodentate Phosphine, Phosphite, Phosphonite, Phosphinite oder bidentate Phosphine, Phosphonite, Phospite, Phosphinite bzw. gemischte bidentate Liganden wie Phosphin/Phosphitkombinationen, bei denen der Phosphor an Aryl- und/oder (Cyclo)alkyl-, Aryloxy- und/oder (Cyclo)alkoxygruppen gebunden ist. Bevorzugte Phosphorliganden sind z.B. Phosphane, Phosphite, Phosphinite oder Phosphonite. Diese Liganden können ein- oder zweizähnige Liganden sein.
Besonders bevorzugte Phosphorliganden sind die nachfolgend aufgeführten Verbindungen: PCy₃, P*ₙ*Bu₃, PPh₃, PAd_{2*n*}Bu, 1,2-Bis(di-*tert*-butylphosphinomethyl)benzol.

Halogenide sind Zusatzstoffe, die auch als Katalysatorliganden fungieren können und die allein oder in Kombination mit einem Lösungsmittel, im erfindungsgemäßen Verfahren eingesetzt werden. Bevorzugt kommen anorganische oder organische Halogenverbindungen zum Einsatz. So können z.B. anorganische Halogenverbindungen der 1. und 2. Hauptgruppe im erfindungsgemäßen Verfahren verwendet werden, wie LiCl, LiBr, LiJ, NaCl, KCl, CsCl oder SnCl₂. Organische Halogenverbindungen, die erfolgreich eingesetzt werden können, sind z.B. Tetraalkylammoniumverbindungen, wie Tetrabutylammoniumchlorid (N*n*Bu₄Cl) und Tetrabutylammoniumbromid (N*n*Bu₄Br), Tetraalkylphosphoniumverbindungen, z. B. Trihexyl(tetradecyl)phosphoniumchlorid , oder Imidazolderivate, wie 1-Alkyl-3-methylimidazoliniumverbindungen, z.B. 1-Butyl-3-methylimidazoliniumchlorid ([Bmim]Cl) oder 1-Ethyl-3-methylimidazoliniumchlorid ([Emim]Cl. Besonders bevorzugt werden im erfindungsgemäßen Verfahren LiCl und/oder [Bmim]Cl eingesetzt.

Ganz besonders bevorzugt wird im erfindungsgemäßen Verfahren ein Katalysatorkomplex umfassend [Ru₃(CO)₁₂], PCy₃ und B(mim)Cl in Kombination mit NMP als Lösungsmittel verwendet.

Erfindungsgemäß werden Formaldehyd und Alkohol vorzugsweise im Überschuss eingesetzt. Überraschender Weise ist Formalin (etwa 35-37 %ige wässrige Lösung des Formaldehyd-Gases, wobei 10-15 Gew.-% Methanol zur Stabilisierung gegen Polymerbildung höherer Aggregate zugesetzt sind) im erfindungsgemäßen Verfahren gänzlich ungeeignet und es kommt zu keiner Esterbildung.

Die Alkene werden vorzugsweise mit der wenigstens einfachen äquivalenten Menge des jeweiligen Alkohols und des Formaldehyds in Gegenwart des Ru-Katalysators umgesetzt.

Die Umsetzung führt zu den entsprechenden Estern in Abhängigkeit vom Alken. Als stöchiometrische Nebenprodukte können in Abhängigkeit vom eingesetzten Alken die jeweilige Alkane in Ausbeuten von bis zu 25% entstehen.

Im erfindungsgemäßen Verfahren erfolgt die Reaktion vorzugsweise bei Temperaturen von 20 bis 160 °C, besonders bevorzugt bei Temperaturen von 80 bis 140 °C. Es muss nicht unter besonderen Druckbedingungen gearbeitet werden. Der Druckbereich liegt vorzugsweise bei Drücken von 1 bis 50 bar, besonders bevorzugt bei Normaldruck der Reaktionsmischung bei Reaktionstemperatur.

Die Reaktionsdauer hängt von der gewählten Temperatur ab. Sie liegt für die weitgehendste Umsetzung des Alkens bevorzugt bei ca. 1 bis 36 Stunden.

Wirksame Katalysatormengen im Verfahren sind vorzugsweise 0,01 bis 10 Mol-% bezogen auf das eingesetzte Alken. Die molaren Verhältnisse Alken : Alkohol können von 1: 75 bis 1:1 variieren. Die Verhältnisse von Ru zu Cl⁻ werden bevorzugt in molaren Verhältnissen von 1 : 331 bis 1 : 8 variieren. Die Volumenverhältnisse von Alkohol zu Lösungsmittel können von 100 : 0 bis 1: 10 variieren. Das Verhältnis von Alken zu Formaledhyd liegt z.B. bei 1-100, bevorzugt 1:10, besonders bevorzugt 1:4.

Das erfindungsgemäße Verfahren ermöglicht auf einfache Weise kostengünstig die Herstellung beliebiger gesättigter Esterverbindungen. Die Reaktion kann in hohen Ausbeuten und mit hoher Selektivität durchgeführt werden. Eine gesonderte Aufarbeitung unter Entfernung von Nebenprodukten kann entfallen.

Es können preiswerte Rutheniumkatalysatoren in Abwesenheit von sauren Cokatalysatoren im erfindungsgemäßen Verfahren unter Verwendung des leicht zu handhabbaren Formaldehyds Verwendung finden. Im Vergleich zu Formiaten kann das erfindungsgemäß eingesetzte Formaldehyd technisch durch die katalytische Oxidation von Methanol, das über CO₂ Hydrierung in großem Maßstab produziert wird, erhalten werden. Außerdem bietet die Nutzung von Formaldehyd die Möglichkeit, die Verwendung von kostenintensiven Hochdruckapparaturen vollständig zu vermeiden. Weiterhin können bei der Verwendung der Kombination von Formaldehyd und Alkoholen verschiedene Ester durch einfache Variation der Alkoholkomponente erhalten werden.

Die Erfindung wird in nachfolgenden Beispielen näher erläutert.

### Ausführungsbeispiele

Abkürzungsverzeichnis:
NMP = *N*-Methyl-2-pyrrolidon
DMA = Dimethylacetamid
[Bmim]CI = 1-Butyl-3-methylimidazoliumchlorid

### Beispiel 1

### Herstellung von Methylcyclohexylcarboxylat

Das folgende Reaktionsschema und Tabelle 1 zeigen die Umsetzung von Cyclohexen, Paraformaldehyd (n=1) und Methanol unter Verwendung diverser Rutheniumkatalysatoren, Liganden und Lösungsmittel zum Methylcyclohexylcarboxylat und ggf. Cyclohexan.

**Tabelle 1:**

| | | | | |
|---|---|---|---|---|
| 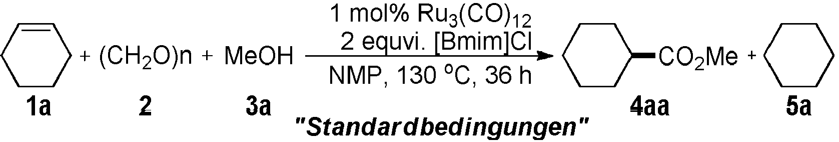 | | | | |

| Nr. | Variation der Standardbebingungen Umsatz% | | Ausbeute%^{[b]} | |
|---|---|---|---|---|
| | | | **4aa** | **5a** |
| 1 | no | 80 | 65 | 2 |
| 2 | Ru(Me-allyl)₂(cod) anstelle Ru₃(CO)₁₂ | 85 | 64 | 2 |
| 4 | LiCl anstelle [Bmim]Cl | 86 | 35 | 6 |
| 5 | KCI anstelle [Bmim]Cl | 60 | 28 | 1 |
| 6 | N*n*Bu₄Cl anstelle [Bmim]Cl | 100 | 49 | 4 |
| 7 | Formalin anstelle Paraformaldehyd | 100 | Spuren | Spuren |
| 8 | DMA anstelle NMP | 77 | 60 | 1 |
| 9 | 1,4-Dioxan anstelle NMP | 30 | 13 | Spuren |
| 10 | Toluen anstelle NMP | 64 | 38 | 2 |
| 11 | 3 mol% PPh₃ zugesetzt | 86 | 54 | 2 |
| 12 | 3 mol% PCy₃ zugesetzt | 89 | 80 | 5 |
| **13**^{[c]} | **4.5 mol% PCy₃ zugesetzt** | **94** | **88** | **Spuren** |
| 14 | 6 mol% PCy₃ zugesetzt | 66 | 50 | Spuren |
| 15 | 3 mol% PAd₂*n*Bu zugesetzt | 90 | 60 | Spuren |
| 16 | 3 mol% L zugesetzt^{[d]} | 90 | 18 | Spuren |

| | | | | |
|---|---|---|---|---|
| [a] Reaktionsbedingungen: **1a** (1 mmol), **2** (120mg, 4 mmol of "CH₂O" Monomer), 1 mol% of Ru₃(CO)₁₂. [Bmim]Cl (2 mmol) in 0.5 mL Methanol und 1.5 mL NMP als Lösungsmittel 130 °C. [b] GC Ausbeuten. [c] 1.5 mol% Ru₃(CO)₁₂. [d] L = 1,2-Bis(di-*tert*-butylphosphinomethyl)benzen. DMA = Dimethylacetamid, NMP = *N*-Methyl-2-pyrrolidon, [Bmim]Cl = 1-Butyl-3-methylimidazoliumchlorid | | | | |

*Beispiel 1.1 (Tabelle 1):* Ein 20-mL Druckrohr wird befüllt mit Ru₃(CO)₁₂ (6.4 mg; 10 µmol), und [Bmim]Cl (0.35 g; 2,00 mmol), Cyclohexen (0.1 mL; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 80% und die Ausbeute an Methylcyclohexylcarboxylat beträgt 65%, Cyclohexan 2%.
*Beispiel 1.2 (Tabelle 1):* Ein 20-mL Druckrohr wird befüllt mit Ru(Me-allyl)₂(cod) (9.6 mg; 30 µmol) und [Bmim]Cl (0.35 g; 2,00 mmol), Cyclohexen (0.1 mL; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 85% und die Ausbeute an Methylcyclohexylcarboxylat beträgt 64%, Cyclohexan 2%.
*Beispiel 1.4 (Tabelle 1):* Ein 20-mL Druckrohr wird befüllt mit Ru₃(CO)₁₂ (6.4 mg; 10 µmol), und LiCl (84 mg; 2,00 mmol), Cyclohexen (0.1 mL; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 86% und die Ausbeute an Methylcyclohexylcarboxylat beträgt 35%, Cyclohexan 6%.
*Beispiel 1.5 (Tabelle 1):* Ein 20-mL Druckrohr wird befüllt mit Ru₃(CO)₁₂ (6.4 mg; 10 µmol), und KCl (148 mg; 2,00 mmol), Cyclohexen (0.1 mL; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 60% und die Ausbeute an Methylcyclohexylcarboxylat beträgt 28%, Cyclohexan 1%.
*Beispiel 1.6 (Tabelle 1):* Ein 20-mL Druckrohr wird befüllt mit Ru₃(CO)₁₂ (6.4 mg; 10 µmol), und N*n*Bu₄Cl (0.54 g; 2,00 mmol), Cyclohexen (0.1 mL; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 100% und die Ausbeute an Methylcyclohexylcarboxylat beträgt 49%, Cyclohexan 4%. (Vergleichsbeispiel)
*Beispiel 1.7 (Tabelle 1):* Ein 20-mL Druckrohr wird befüllt mit Ru₃(CO)₁₂ (6.4 mg; 10 µmol), und [Bmim]Cl (0.35 g; 2,00 mmol), Cyclohexen (0.1 mL; 1.0 mmol), MeOH (0.5 mL) und NMP (1.5 mL) werden hinzugegeben und es wird Formalin (0.3 mL; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 100% und die Ausbeute an Methylcyclohexylcarboxylat beträgt 0%, Cyclohexan 0%.
*Beispiel 1.8 (Tabelle 1):* Ein 20-mL Druckrohr wird befüllt mit Ru₃(CO)₁₂ (6.4 mg; 10 µmol), und [Bmim]Cl (0.35 g; 2,00 mmol), Cyclohexen (0.1 mL; 1.0 mmol), MeOH (0.5 mL) und DMA (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 77% und die Ausbeute an Methylcyclohexylcarboxylat beträgt 60%, Cyclohexan 1%.
*Beispiel 1.9 (Tabelle 1):* Ein 20-mL Druckrohr wird befüllt mit Ru₃(CO)₁₂ (6.4 mg; 10 µmol), und [Bmim]Cl (0.35 g; 2,00 mmol), Cyclohexen (0.1 mL; 1.0 mmol), MeOH (0.5 mL) und 1,4 Dioxane (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 30% und die Ausbeute an Methylcyclohexylcarboxylat beträgt 13%, Cyclohexan 0%.
*Beispiel 1.10 (Tabelle 1):* Ein 20-mL Druckrohr wird befüllt mit Ru₃(CO)₁₂ (6.4 mg; 10 µmol), und [Bmim]Cl (0.35 g; 2,00 mmol), Cyclohexen (0.1 mL; 1.0 mmol), MeOH (0.5 mL) und Toluene (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 64% und die Ausbeute an Methylcyclohexylcarboxylat beträgt 38%, Cyclohexan 2%.
*Beispiel 1.11 (Tabelle 1):* Ein 20-mL Druckrohr wird befüllt mit Ru₃(CO)₁₂ (6.4 mg; 10 µmol), Triphenylphosphin (7.9 mg; 30 µmol) und [Bmim]Cl (0.35 g; 2,00 mmol), Cyclohexen (0.1 mL; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 86% und die Ausbeute an Methylcyclohexylcarboxylat beträgt 54%, Cyclohexan 2%.
*Beispiel 1.12 (Tabelle 1):* Ein 20-mL Druckrohr wird befüllt mit Ru₃(CO)₁₂ (6.4 mg; 10 µmol), Tricyclohexylphosphin (8.4 mg; 30 µmol) und [Bmim]Cl (0.35 g; 2,00 mmol), Cyclohexen (0.1 mL; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 89% und die Ausbeute an Methylcyclohexylcarboxylat beträgt 80%, Cyclohexan 5%.
*Beispiel 1.13 (Tabelle 1):* Ein 20-mL Druckrohr wird befüllt mit Ru₃(CO)₁₂ (9.6 mg; 15 µmol), Tricyclohexylphosphin (12.6 mg; 45 µmol) und [Bmim]Cl (0.35 g; 2,00 mmol), Cyclohexen (0.1 mL; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 94% und die Ausbeute an Methylcyclohexylcarboxylat beträgt 88%, Cyclohexan 0%.
*Beispiel 1.14 (Tabelle 1):* Ein 20-mL Druckrohr wird befüllt mit Ru₃(CO)₁₂ (6.4 mg; 10 µmol), Tricyclohexylphosphin (16.8 mg; 60 µmol) und [Bmim]Cl (0.35 g; 2,00 mmol), Cyclohexen (0.1 mL; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühl. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 66% und die Ausbeute an Methylcyclohexylcarboxylat beträgt 50%, Cyclohexan 0%.
*Beispiel 1.15 (Tabelle 1):* Ein 20-mL Druckrohr wird befüllt mit Ru₃(CO)₁₂ (6.4 mg; 10 µmol), cataCXium® A (10.7 mg; 30 µmol) und [Bmim]Cl (0.35 g; 2,00 mmol), Cyclohexen (0.1 mL; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 90% und die Ausbeute an Methylcyclohexylcarboxylat beträgt 60%, Cyclohexan 0%.
*Beispiel 1.16 (Tabelle 1):* Ein 20-mL Druckrohr wird befüllt mit Ru₃(CO)₁₂ (6.4 mg; 10 µmol), 1,2-Bis(di-*tert*-butylphosphinomethyl)benzen (11.8 mg; 30 µmol) und [Bmim]Cl (0.35 g; 2,00 mmol), Cyclohexen (0.1 mL; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 90% und die Ausbeute an Methylcyclohexylcarboxylat beträgt 18%, Cyclohexan 0%.

### Beispiel 2

### Herstellung von diversen Methylcarboxylaten

Das folgende Reaktionsschema und Tabelle 2 zeigen die Umsetzung von diversen Alkenen, Paraformaldehyd (n=1) und Methanol.

**Tabelle 2:**

| | | | | |
|---|---|---|---|---|
| 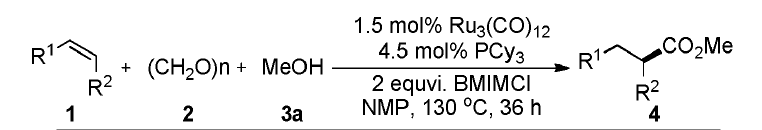 | | | | |

| Nr. | 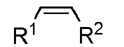 | **1** | Umsatz% | Ausbeute%^{[b]}(n:i) |
|---|---|---|---|---|
| 18 | 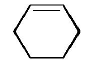 | **1a** | 94 | 88 |
| 19 | 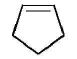 | **1b** | 100 | 90 |
| 20 | 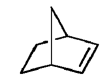 | **1c** | 96 | 82 |
| 21^{[c]} | 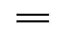 | **1d** | n.d | 51 |
| 22 | 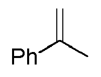 | **1e** | 59 | 51^{[d]} |
| 23 | 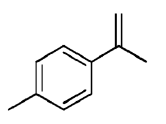 | **1f** | 56 | 50^{[d]} |
| 24 | 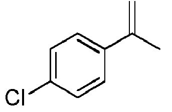 | **1g** | 74 | 68^{[d]} |
| 25 | 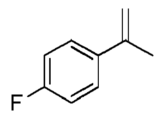 | **1h** | 69 | 62^{[d]} |
| 26 | 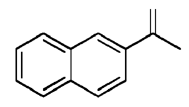 | **1i** | 60 | 53^{[d]} |
| 27 | 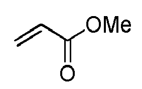 | **1j** | 100 | 43 |
| 28^{[e]} | 1-Octen | **1k** | 100 | 74 (51:49) |
| 29^{[e]} | 2-Octen | **1l** | 100 | 70 (50:50) |

| | | | | |
|---|---|---|---|---|
| a] Reaktionsbedingungen: **1** (1 mmol), **2** (120 mg, 4 mmol of "CH₂O" Monomer), 1.5 mol% Ru₃(CO)₁₂, 4.5 mol% of PCy₃, BIMIMCl (2 mmol) in 0.5 mL Methanol und 1.5 mL NMP als Lösungsmittel bei 130 °C. [b] GC Ausbeuten. [c] **1** (4.2 mmol), **2** (480 mg, 16 mmol of "CH₂O" Monomer), 0.7 mol% of Ru₃(CO)₁₂, BIMIMCl (8 mmol) in 2 mL Methanol und 4 mL NMP als Lösungsmittel bei 130 °C. [d] isolierte Ausbeuten. [e] 0.5 mol% Ru₃(CO)₁₂ ohne PCy₃. | | | | |

*Beispiel 2.18 (Tabelle 2):* Ein 20-mL Druckrohr wird befüllt mit Ru₃(CO)₁₂ (9.6 mg; 15 µmol), Tricyclohexylphosphin (12.6 mg; 45 µmol) und [Bmim]Cl (0.35 g; 2,00 mmol), Cyclohexen (0.1 mL; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 94% und die Ausbeute an Methylcyclohexylcarboxylat beträgt 88%.
*Beispiel 2.19 (Tabelle 2):* Ein 20-mL Druckrohr wird befüllt mit Ru₃(CO)₁₂ (9.6 mg; 15 µmol), Tricyclohexylphosphin (12.6 mg; 45 µmol) und [Bmim]CI (0.35 g; 2,00 mmol), Cyclopenten (0.088 mL; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclopenten beträgt 100% und die Ausbeute an Methylcyclopentylcarboxylat beträgt 90%.
*Beispiel 2.20 (Tabelle 2):* Ein 20-mL Druckrohr wird befüllt mit Ru₃(CO)₁₂ (9.6 mg; 15 µmol), Tricyclohexylphosphin (12.6 mg; 45 µmol) und [Bmim]Cl (0.35 g; 2,00 mmol), Bicyclo[2.2.1]hept-2-en (94 mg; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Bicyclo[2.2.1]hept-2-en beträgt 96% und die Ausbeute an Methylbicyclo[2.2.1]heptyl-2-carboxylat beträgt 82%.
*Beispiel 2.21 (Tabelle 2):* Ein 20-mL Druckrohr wird befüllt mit Ru₃(CO)₁₂ (19,3 mg; 30 µmol), [Bmim]CI (1,4 g; 8,00 mmol), Ethylen (100 mg; 4,2 mmol), MeOH (2 mL) und NMP (4 mL) werden hinzugegeben und es wird Formaldehyd (480 mg; 16.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.3 mL; 207 mg; 1.8 mmol) wird die Reaktionsmischung gaschromatographisch analysiert. Die Ausbeute an C3-Ester beträgt 51%.
*Beispiel 2.22 (Tabelle 2):* Ein 20-mL Druckrohr wird befüllt mit Ru₃(CO)₁₂ (9.6 mg; 15 µmol), Tricyclohexylphosphin (12.6 mg; 45 µmol) und [Bmim]Cl (0.35 g; 2,00 mmol), Isopropenylbenzol (0.13 mL; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Isopropenylbenzol beträgt 59% und die Ausbeute an Methyl-3-phenylbutyrat beträgt 51%.
*Beispiel 2.23 (Tabelle 2):* Ein 20-mL Druckrohr wird befüllt mit Ru₃(CO)₁₂ (9.6 mg; 15 µmol), Tricyclohexylphosphin (12.6 mg; 45 µmol) und [Bmim]Cl (0.35 g; 2,00 mmol), 1-Isopropenyl-4-Methylbenzol (0.15 mL; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von 1-Isopropenyl-4-methylbenzol beträgt 56% und die Ausbeute an Methyl-3 - (*p*-tolyl) butan beträgt 50%.
*Beispiel 2.24 (Tabelle 2):* Ein 20-mL Druckrohr wird befüllt mit Ru₃(CO)₁₂ (9.6 mg; 15 µmol), Tricyclohexylphosphin (12.6 mg; 45 µmol) und [Bmim]Cl (0.35 g; 2,00 mmol), 1-Chlor-4-isopropenylbenzol (0.14 mL; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von 1-Chlor-4-Isopropenylbenzol beträgt 74% und die Ausbeute an Methyl-3-(4-chlorphenyl)butanoat beträgt 68%.
*Beispiel 2.25 (Tabelle 2):* Ein 20-mL Druckrohr wird befüllt mit Ru₃(CO)₁₂ (9.6 mg; 15 µmol), Tricyclohexylphosphin (12.6 mg; 45 µmol) und [Bmim]Cl (0.35 g; 2,00 mmol), 1-Fluor-4-isopropenylbenzol (140 mg; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von 1-Fluor-4-isopropenylbenzol beträgt 69% und die Ausbeute an Methyl-3-(4-fluor-phenyl)butanoat beträgt 62%.
*Beispiel 2.26 (Tabelle 2):* Ein 20-mL Druckrohr wird befüllt mit Ru₃(CO)₁₂ (9.6 mg; 15 µmol), Tricyclohexylphosphin (12.6 mg; 45 µmol) und [Bmim]Cl (0.35 g; 2,00 mmol), 2-Isopropenylnaphthalin (168 mg; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Isopropenylnaphthalin beträgt 60% und die Ausbeute an Methyl-3-(naphthalen-2-yl)butanoat beträgt 53%.
*Beispiel 2.27 (Tabelle 2):* Ein 20-mL Druckrohr wird befüllt mit Ru₃(CO)₁₂ (9.6 mg; 15 µmol), Tricyclohexylphosphin (12.6 mg; 45 µmol) und [Bmim]Cl (0.35 g; 2,00 mmol), 2-Methylmethacrylat (0.1 mL; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Methylmethacrylat beträgt 100% und die Ausbeute an Dimethylmethylsuccinat beträgt 43%.
*Beispiel 2.28 (Tabelle 2):* Ein 20-mL Druckrohr wird befüllt mit Ru₃(CO)₁₂ (3.2 mg; 5 µmol), und [Bmim]Cl (0.35 g; 2,00 mmol), 1-Octen (0.16 mL; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von 1-Octen beträgt 100% und die Ausbeute an C-9 Ester beträgt 74% mit einem *n*/*i*-Verhältnis von 51:49.
*Beispiel 2.29 (Tabelle 2):* Ein 20-mL Druckrohr wird befüllt mit Ru₃(CO)₁₂ (3.2 mg; 5 µmol), und [Bmim]CI (0.35 g; 2,00 mmol), 2-Octen (0.16 mL; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von 2-Octen beträgt 100% und die Ausbeute an C-9 Ester beträgt 70% mit einem *n*/*i*-Verhältnis von 50:50.

### Beispiel 3

### Herstellung von diversen Cyclohexylcarboxylaten

Das folgende Reaktionsschema und Tabelle 3 zeigen die Umsetzung von Cyclohexen, Paraformaldehyd (n=1) und diversen Alkoholen.

**Tabelle 3:**

| | | | | |
|---|---|---|---|---|
| 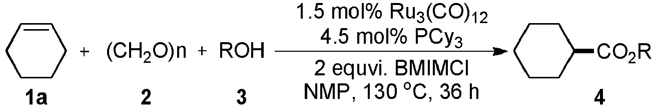 | | | | |

| Nr. | ROH | **3** | Umsatz% | Ausbeute%^{[b]} |
|---|---|---|---|---|
| 30 | MeOH | **3a** | 94 | 88 |
| 31 | EtOH | **3b** | 88 | 78 |
| 32 | *n*BuOH | **3c** | 80 | 69 |
| 33^{[c]} | *n*C₉H₁₉OH | **3d** | 65 | 55 |
| 34 | 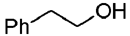 | **3e** | 75 | 68 |
| 35 | 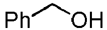 | **3f** | 76 | 71 |
| 36^{[c]} | 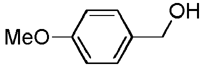 | **3g** | 61 | 50 |
| 37^{[c]} | 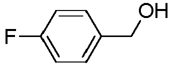 | **3h** | 72 | 61 |
| 38 | 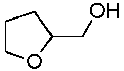 | **3i** | 72 | 62 |
| 39 | *i*PrOH | **3j** | 56 | 40 |

| | | | | |
|---|---|---|---|---|
| [a] Reaktionsbedingungen: **1a** (1 mmol), **2** (120 mg, 4 mmol of "CH₂O" Monomer), 1.5 mol% Ru₃(CO)₁₂, 4.5 mol% PCy₃, BIMIMCl (2 mmol) in 0.5 mL ROH und 1.5 mL NMP als Lösungsmittel bei 130 °C. [b] GC Ausbeuten. [c] 1 mL ROH und 1 mL NMP als Lösungsmittel | | | | |

*Beispiel 3.30 (Tabelle 3):* Ein 20-mL Druckrohr wird befüllt mit Ru₃(CO)₁₂ (9.6 mg; 15 µmol), Tricyclohexylphosphin (12.6 mg; 45 µmol) und [Bmim]Cl (0.35 g; 2,00 mmol), Cyclohexen (0.1 mL; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 94% und die Ausbeute an Methylcyclohexylcarboxylat beträgt 88%.
*Beispiel 3.31 (Tabelle 3):* Ein 20-mL Druckrohr wird befüllt mit Ru₃(CO)₁₂ (9.6 mg; 15 µmol), Tricyclohexylphosphin (12.6 mg; 45 µmol) und [Bmim]Cl (0.35 g; 2,00 mmol), Cyclohexen (0.1 mL; 1.0 mmol), Ethanol (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 88% und die Ausbeute an Ethylcyclohexylcarboxylat beträgt 78%.
*Beispiel 3.32 (Tabelle 3):* Ein 20-mL Druckrohr wird befüllt mit Ru₃(CO)₁₂ (9.6 mg; 15 µmol), Tricyclohexylphosphin (12.6 mg; 45 µmol) und [Bmim]Cl (0.35 g; 2,00 mmol), Cyclohexen (0.1 mL; 1.0 mmol), Butanol (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 80% und die Ausbeute an Butylcyclohexylcarboxylat beträgt 69%.
*Beispiel 3.33 (Tabelle 3):* Ein 20-mL Druckrohr wird befüllt mit Ru₃(CO)₁₂ (9.6 mg; 15 µmol), Tricyclohexylphosphin (12.6 mg; 45 µmol) und [Bmim]Cl (0.35 g; 2,00 mmol), Cyclohexen (0.1 mL; 1.0 mmol), Nonanol (1 mL) und NMP (1 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 65% und die Ausbeute an Nonanoylcyclohexylcarboxylat beträgt 55%.
*Beispiel 3.34 (Tabelle 3):* Ein 20-mL Druckrohr wird befüllt mit Ru₃(CO)₁₂ (9.6 mg; 15 µmol), Tricyclohexylphosphin (12.6 mg; 45 µmol) und [Bmim]Cl (0.35 g; 2,00 mmol), Cyclohexen (0.1 mL; 1.0 mmol), 2-Phenylethanol (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 75% und die Ausbeute an Phenethylcyclohexylcarboxylat beträgt 68%.
*Beispiel 3.35 (Tabelle 3):* Ein 20-mL Druckrohr wird befüllt mit Ru₃(CO)₁₂ (9.6 mg; 15 µmol), Tricyclohexylphosphin (12.6 mg; 45 µmol) und [Bmim]Cl (0.35 g; 2,00 mmol), Cyclohexen (0.1 mL; 1.0 mmol), Benzylalkohol (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 76% und die Ausbeute an Benzylcyclohexylcarboxylat beträgt 71%.
*Beispiel 3.36 (Tabelle 3):* Ein 20-mL Druckrohr wird befüllt mit Ru₃(CO)₁₂ (9.6 mg; 15 µmol), Tricyclohexylphosphin (12.6 mg; 45 µmol) und [Bmim]Cl (0.35 g; 2,00 mmol), Cyclohexen (0.1 mL; 1.0 mmol), Anisylalkohol (1.0 mL) und NMP (1 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 61% und die Ausbeute an 4-Methoxybenzylcyclohexylcarboxylat beträgt 50%.
*Beispiel 3.37 (Tabelle 3):* Ein 20-mL Druckrohr wird befüllt mit Ru₃(CO)₁₂ (9.6 mg; 15 µmol), Tricyclohexylphosphin (12.6 mg; 45 µmol) und [Bmim]Cl (0.35 g; 2,00 mmol), Cyclohexen (0.1 mL; 1.0 mmol), 4-Fluorobenzylalkohol (1.0 mL) und NMP (1 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 72% und die Ausbeute an 4-Fuorbenzylcyclohexylcarboxylat beträgt 61%.
*Beispiel 3.38 (Tabelle 3):* Ein 20-mL Druckrohr wird befüllt mit Ru₃(CO)₁₂ (9.6 mg; 15 µmol), Tricyclohexylphosphin (12.6 mg; 45 µmol) und [Bmim]Cl (0.35 g; 2,00 mmol), Cyclohexen (0.1 mL; 1.0 mmol), Tetrahydrofurfurylalkohol (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 72% und die Ausbeute an (Tetrahydrofuran-2-yl)methylcyclohexylcarboxylat beträgt 62%.
*Beispiel 3.39 (Tabelle 3):* Ein 20-mL Druckrohr wird befüllt mit Ru₃(CO)₁₂ (9.6 mg; 15 µmol), Tricyclohexylphosphin (12.6 mg; 45 µmol) und [Bmim]Cl (0.35 g; 2,00 mmol), Cyclohexen (0.1 mL; 1.0 mmol), Isopropanol (0.5 mL) und NMP (1.5 mL) werden hinzugegeben und es wird Formaldehyd (120 mg; 4.0 mmol) zugesetzt. Die Reaktionsmischung wird auf 130 °C erwärmt und 40 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 56% und die Ausbeute an (Tetrahydrofuran-2-yl)methylcyclohexylcarboxylat beträgt 40%.

### Beispiel 4

### Herstellung von Methylcyclohexylcarboxylat

Das folgende Reaktionsschema und Tabelle 4 zeigen die Umsetzung von Cyclohexen, Paraformaldehyd (n=1) und Methanol unter Verwendung Ru3(CO)12, PCy3 B(mim)Cl und NMP.

**Tabelle 4:**

| | | |
|---|---|---|
| 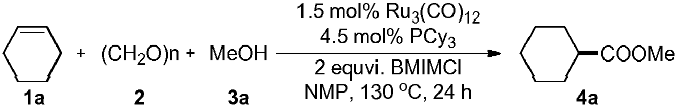 | | |

| Nr. | Umsatz% | Ausbeute% |
|---|---|---|
| 40 | 85 | 82 |
| 41 | 79 | 73 |
| 42 | 74 | 69 |
| 43 | 66 | 63 |

*Beispiel 4.40 (Tabelle 4):* Ein 20-mL Druckrohr wird befüllt mit Ru₃(CO)₁₂ (9.6 mg; 15 µmol), Tricyclohexylphosphin (12.6 mg; 45 µmol) und [Bmim]Cl (0.35 g; 2,00 mmol), Cyclohexen (0.1 mL; 1.0 mmol), MeOH (0.5 mL) und NMP (1.5 mL) werden hinzugegeben und es wird Formaldehyd (120 mg; 4.0 mmol) zugesetzt. Die Reaktionsmischung wird auf 130 °C erwärmt und 24 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 85% und die Ausbeute an Methylcyclohexylcarboxylat beträgt 82%.
*Beispiel 4.41 (Tabelle 4):* Ein 20-mL Druckrohr wird befüllt mit Cyclohexen (0.1 mL; 1.0 mmol), MeOH (0.5 mL) und die Reaktionslösung von Beispiel 40 werden hinzugegeben und es wird Formaldehyd (120 mg; 4.0 mmol) zugesetzt. Die Reaktionsmischung wird auf 130 °C erwärmt und 24 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 79% und die Ausbeute an Methylcyclohexylcarboxylat beträgt 73%.
*Beispiel 4.42 (Tabelle 4):* Ein 20-mL Druckrohr wird befüllt mit Cyclohexen (0.1 mL; 1.0 mmol), MeOH (0.5 mL) und die Reaktionslösung von Beispiel 41 werden hinzugegeben und es wird Formaldehyd (120 mg; 4.0 mmol) zugesetzt. Die Reaktionsmischung wird auf 130 °C erwärmt und 24 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 74% und die Ausbeute an Methylcyclohexylcarboxylate beträgt 69%.
*Beispiel 4.43 (Tabelle 4):* Ein 20-mL Druckrohr wird befüllt mit Cyclohexen (0.1 mL; 1.0 mmol), MeOH (0.5 mL) und die Reaktionslösung von Beispiel 42 werden hinzugegeben und es wird Formaldehyd (120 mg; 4.0 mmol) zugesetzt. Die Reaktionsmischung wird auf 130 °C erwärmt und 24 Stunden bei dieser Temperatur gerührt. Danach wird das Druckrohr abgekühlt. Nach der Zugabe des internen Standards (Isooktan, 0.1 mL; 70 mg; 0.6 mmol) wird die Reaktionsmischung gaschromatographisch analysiert. Der Umsatz von Cyclohexen beträgt 66% und die Ausbeute an Methylcyclohexylcarboxylat beträgt 63%.

## Patentansprüche

1. Verfahren zur katalytischen Herstellung von Estern durch Alkoxycarbonylierung,
**dadurch gekennzeichnet, dass** mindestens ein Alken mit Formaldehyd und mindestens einem Alkohol in Gegenwart eines Rutheniumkatalysators umgesetzt wird,
wobei Alkene, die im erfindungsgemäßen Verfahren als Ausgangsmaterialien fungieren, können einfach oder mehrfach ungesättigte lineare n-Alkene oder verzweigte Isoalkene, Cycloalkene und aromatische Alkene mit einer Kohlenstoffzahl bevorzugt von 2 bis 40 sein, die gegebenenfalls mit Ester-, Aryl-, Nitro-, Hydroxyl-, Nitril-, Ethergruppen oder Halogenatomen substituiert sind;
wobei Formaldehyde Paraformaldehyd der allgemeinen Formel [CH₂O]ₙ bedeutet, wobei n = 1 bis 200 sein kann, und wobei das Paraformaldehyd festes Formaldehyd-Polymer bezeichnet, das eine Mischung von kurzkettigen, linearen Poly(oxymethylen)en enthält, und der Katalysator ein Komplex aus Ru₃(CO)₁₂ oder Ru(Me-allyl)₂(COD) ist, gegebenenfalls in Kombination mit einem Halogenid und/oder einer trivalenten organischen Phosphorverbindung.

2. Verfahren nach Anspruch 1,
**dadurch gekennzeichnet, dass** der Rutheniumkatalysator ein Rutheniumkomplex ist, der durch mindestens einen Liganden koordiniert ist, wobei die Liganden ausgewählt sind aus der Gruppe umfassend CO, Halogenide, Tetrafluoroborat, Amide, Oxide, Carbanionen, Carboxylate, Hydride, Sulfate und/oder trivalente organische Phosphorverbindungen.

3. Verfahren nach Anspruch 2,
**dadurch gekennzeichnet, dass** die Phosphorverbindungen monodentate Phosphine, Phosphite, Phosphonite, Phosphinite oder bidentate Phosphine, Phosphonite, Phospite, Phosphinite bzw. gemischte bidentate Liganden wie Phosphin/Phosphitkombinationen sind, bei denen der Phosphor an Aryl- und/oder (Cyclo)alkyl-, Aryloxy- und/oder (Cyclo)alkoxygruppen gebunden ist.

4. Verfahren nach einem der Ansprüche 2 oder 3,
**dadurch gekennzeichnet, dass** das Halogenid ein anorganisches Halogenid ist, vorzugsweise ausgewählt aus der Gruppe LiCl, LiBr, Lil, NaCl, KCl, CsCl, SnCl₂ und Gemischen davon; und/oder ein organisches Halogenid ist, vorzugsweise ausgewählt aus der Gruppe der Tetraalkylammoniumverbindungen, Phosphoniumverbindungen oder Imidazolderivaten.

5. Verfahren nach einem der Ansprüche 1 bis 4,
**dadurch gekennzeichnet, dass** die Reaktion in Gegenwart eines Lösungsmittels durchgeführt wird.

6. Verfahren nach einem der Ansprüche 1 bis 5,
**dadurch gekennzeichnet, dass** die Reaktionstemperatur bei 20 bis 160°C liegt, bevorzugt bei 80 bis 140°C.

7. Verfahren nach einem der Ansprüche 1 bis 6,
**dadurch gekennzeichnet, dass** der Druck im Bereich von 1 bis 50 bar liegt, vorzugsweise Normaldruck.

## Claims

1. Method for the catalytic preparation of esters by alkoxycarbonylation, **characterized in that** at least one alkene is reacted with formaldehyde and at least one alcohol in the presence of a ruthenium catalyst, wherein alkenes, which function as starting materials in the method according to the invention, may be mono- or polyunsaturated linear n-alkenes or branched isoalkenes, cycloalkenes and aromatic alkenes having a carbon number preferably of 2 to 40, which are optionally substituted with ester, aryl, nitro, hydroxyl, nitrile or ether groups or halogen atoms; wherein formaldehydes signifies paraformaldehyde of the general formula [CH₂O]ₙ, where n can be from 1 to 200, and wherein the paraformaldehyde refers to solid formaldehyde polymer which comprises a mixture of short-chain, linear poly(oxymethylene)s, and the catalyst is a complex of Ru3(CO)12 or Ru(Me-allyl)2(COD), optionally in combination with a halide and/or a trivalent organic phosphorus compound.

2. Method according to Claim 1, **characterized in that** the ruthenium catalyst is a ruthenium complex coordinated with at least one ligand, wherein the ligands are selected from the group comprising CO, halides, tetrafluoroborate, amides, oxides, carbanions, carboxylates, hydrides, sulphates and/or trivalent organic phosphorus compounds.

3. Method according to Claim 2, **characterized in that** the phosphorus compounds are monodentate phosphines, phosphites, phosphonites, phosphinites or bidentate phosphines, phosphonites, phosphites, phosphinites or mixed bidentate ligands such as phosphine/phosphite combinations, in which the phosphorus is bonded to aryl and/or (cyclo)alkyl, aryloxy and/or (cyclo)alkoxy groups.

4. Method according to either of Claims 2 and 3, **characterized in that** the halide is an inorganic halide, preferably selected from the group of LiCl, LiBr, LiI, NaCl, KC1, CsCl, SnCl₂ and mixtures thereof; and/or is an organic halide, preferably selected from the group of tetraalkylammonium compounds, phosphonium compounds or imidazole derivatives.

5. Method according to any of Claims 1 to 4, **characterized in that** the reaction is carried out in the presence of a solvent.

6. Method according to any of Claims 1 to 5, **characterized in that** the reaction temperature is 20 to 160°C, preferably 80 to 140°C.

7. Method according to any of Claims 1 to 6, **characterized in that** the pressure is in the range of 1 to 50 bar, preferably standard pressure.

## Revendications

1. Procédé pour la préparation catalytique d'esters par alcoxycarbonylation, **caractérisé en ce qu'**au moins un alcène est transformé avec du formaldéhyde et au moins un alcool en présence d'un catalyseur à base de ruthénium,
les alcènes, qui fonctionnent dans le procédé selon l'invention comme matériaux de départ, pouvant être des n-alcènes linaires ou des isoalcènes ramifiés, des cycloalcènes, mono-insaturés ou polyinsaturés, et des alcènes aromatiques présentant un nombre d'atomes de carbone de préférence de 2 à 40, qui sont le cas échéant substitués par des groupes ester, aryle, nitro, hydroxyle, nitrile, éther ou des atomes d'halogène ; formaldéhyde signifiant le paraformaldéhyde de formule générale [CH₂O]ₙ, dans laquelle n = 1 à 200, et le paraformaldéhyde désignant un polymère solide de formaldéhyde qui contient un mélange de poly(oxyméthylènes) linéaires à courte chaîne,
et le catalyseur étant un complexe de Ru₃(CO)₁₂ ou de Ru(Me-allyle)₂(COD), le cas échéant en combinaison avec un halogénure et/ou avec un composé phosphoré organique trivalent.

2. Procédé selon la revendication 1, **caractérisé en ce que** le catalyseur de ruthénium est un complexe du ruthénium, qui est coordiné par au moins un ligand, les ligands étant choisis dans le groupe comprenant CO, les halogénures, le tétrafluoroborate, les amides, les oxydes, les carbanions, les carboxylates, les hydrures, les sulfates et/ou les composés phosphorés organiques trivalents.

3. Procédé selon la revendication 2, **caractérisé en ce que** les composés phosphorés sont des phosphines, des phosphites, des phosphonites, des phosphinites monodentates ou des phosphines, des phosphonites, des phosphites, des phosphinites bidentates ou, selon le cas, des ligands bidentates mixtes tels que des combinaisons phosphine/phosphite, dans lesquels le phosphore est lié à des groupes aryle et/ou (cyclo)alkyle, aryloxy et/ou (cyclo)alcoxy.

4. Procédé selon l'une quelconque des revendications 2 ou 3, **caractérisé en ce que** l'halogénure est un halogénure inorganique, de préférence choisi dans le groupe formé par LiCl, LiBr, LiI, NaCl, KCl, CsCl, SnCl₂ et leurs mélanges ; et/ou est un halogénure organique, de préférence choisi dans le groupe des composés de tétraalkylammonium, de phosphonium ou des dérivés d'imidazole.

5. Procédé selon l'une quelconque des revendications 1 à 4, **caractérisé en ce que** la réaction est réalisée en présence d'un solvant.

6. Procédé selon l'une quelconque des revendications 1 à 5, **caractérisé en ce que** la température de réaction est de 20 à 160°C, de préférence de 80 à 140°C.

7. Procédé selon l'une quelconque des revendications 1 à 6, **caractérisé en ce que** la pression se situe dans la plage de 1 à 50 bars, de préférence à la pression normale.
